# EUROPEAN PATENT SPECIFICATION

(11) **EP 2 070 547 B1**
(45) Date of publication and mention of the grant of the patent: **24.04.2013**
(21) Application number: 07817381.2
(22) Date of filing: 27.09.2007
(51) Int. Cl.: A61K 39/395, A61K 38/21, A61P 35/00

(54) **A COMPOSITION COMPRISING HUMANIZED ANTI-EGFR MAB H-R3 ANTIBODY AND TYPE I INF FOR USE IN TREATING CANCER**
EINE ZUSAMMENSETZUNG ENTHALTEND HUMANISIERTEN ANTIKÖRPER ANTI-EGFR MAB H-R3 UND TYP I NF IN VERWENDUNG ZUR BEHANDLUNG VON KREBS
UNE COMPOSITION COMPRENANT HUMANISÉ ANTI-EGFR MAB H-R3 ANTICORPS ET L'INF DE TYPE I POUR UNE UTILISATION DANS LE TRAITEMENT DU CANCER

(30) Priority: 29.09.2006 CU 20060190
(43) Date of publication of application: 17.06.2009
(73) Proprietor: Centro de Inmunologia Molecular, Ciudad de la Habana 12100 (CU)
(72) Inventor: FERNÁNDEZ MOLINA, Luis Enrique, Ciudad De La Habana 11600 (CU); GARRIDO HIDALGO, Greta, Ciudad De La Habana (CU); PÉREZ RODRÍGUEZ, Rolando, Ciudad De La Habana (CU); SÁNCHEZ RAMÍREZ, Belinda, Ciudad De La Habana 11600 (CU); FERNÁNDEZ GÓMEZ, Audry, Ciudad De La Habana 11600 (CU); LÓPEZ REQUENA, Alejandro, Ciudad De La Habana 10500 (CU); BEAUSOLEIL DELGADO, Irene, Ciudad De La Habana 11600 (CU)
(74) Representative: Hatzmann, Martin
(86) International application number: PCT/CU2007/000017
(87) International publication number: WO 2008/037225

(56) References cited:
- EP-A1- 0 712 863
- WO-A1-02/089842
- US-A1- 2006 134 064
- US-A1- 2006 134 064
- RAMOS T.C. ET AL.: 'Treatment of high-grade glioma patients with the humanized anti-epidermal growth factor receptor (EGFR) antibody h-R3: report from a phase I/II trial' CANCER BIOL. THER. vol. 5, no. 4, April 2006, pages 375 - 379, XP008104828
- CROMBET T. ET AL.: 'Use of the Humanized Anti-Epidermal Growth Factor Receptor Monoclonal Antibody h-R3 in Combination with Advanced Head and Neck Cancer Patients' J. CLIN ONCOL. vol. 22, no. 9, 2004, pages 1646 - 1654, XP008131473
- BRUZZESE F. ET AL.: 'Synergistic antitumor activity of epidermal growth factor receptor tyrosine kinase inhibitor gefitinib and IFN-alpha in head and neck cancer cells in vitro and in vivo' CLIN. CANCER RES. vol. 12, no. 2, January 2006, pages 617 - 625, XP008104824
- YANG J.L. ET AL.: 'Interferon-alpha promotes the anti-proliferative effect of gefitinib (ZD 1839) on human colon cancer cell lines' ONCOLOGY vol. 69, no. 3, 2005, pages 224 - 238, XP008131550
- YANG J.L. ET AL.: 'Interferon-alpha promotes the anti-proliferative effect of Erlotinib (OSI-774) on human colon cancer cell lines' CANCER LETT. vol. 225, no. 1, 2005, pages 61 - 74, XP004939075
- CROMBET-RAMOS T. ET AL.: 'Antiproliferative angiogenetic and proapoptotic activity of h-R3: A humanized anti-EGFR antibody' INT. J. CANCER vol. 101, no. 6, 2002, pages 567 - 575, XP008104826
- GRONWALD V. ET AL.: 'Developing inhibitors of the epidermal growth factor receptor for cancer treatment' J. NATL. CANCER INST. vol. 95, no. 12, 2003, pages 851 - 867, XP008048667

## Description

### Filed of the invention

The present invention relates to the biotechnological field, particularly with the specific cancer immunotherapy. The present invention is based on the synergic effect on the metastasis growth of the combination of anti-Epidermal Growth Factor Receptor monoclonal antibodies (anti-EGFR Mabs) and type I Interferons (IFNs). Thus, the present invention provides a therapeutic tool that overcomes the limitations of anti-EGFR monotherapies.

### Prior Art

### Anti-EGFR monoclonal antibodies

The EGFR and its ligands are expressed in normal tissues with the exception of haematopoietic cells (Carpenter G. Annu Rev Biochem 1987; 56:881-914). The over expression of these proteins has been detected in many human epithelial tumors (Salomon DS et al. Crit Rev Oncol Hematol 1995; 19:183-232*).* Pre-clinical studies have demonstrated that EGFR-ligands autocrine and paracrine loops regulate proliferation and tumor cells metastatic capacity *(*Verbeek BS et al. FEBS Lett 1998; 425:145-50*;* O-Charoenrat P et al. Int J Cancer 2000; 86:307-17; Radinsky R et al. Clin 20 Cancer Res 1995; 1:19-31*).* As a result, powerful and selective EGFR antagonists are currently in clinical trials *(*Pal SK, Pegram M. Anticancer Drugs 2005; 16:483-94*).*

Nowadays, the most successful therapy in clinical development is the chimeric Mab IMC-C225/Cetuximab. Cetuximab binds to subdomain III of the extracellular domain of the receptor, competes with the ligand and blocks activation of receptor by affecting receptor dimerization. Also, Cetuximab induces the internalization and degradation of EGFR (Shiqing L et al. Cancer Cell 2005; 7:301-11*)*. In addition, Naramura and coworkers demonstrated that Cetuximab can induce antibody-dependent cellular cytotocixity (ADCC) through the activation of patient periphery blood mononuclear cells, suggesting that this mechanism could contribute to the anti-tumor activity of this anti-EGFR Mab (Naramura M et al. Cancer Immunol Immunother 1993; 37:343-9). Pre-clinical studies using Cetuximab, have rendered complete regressions of human xenograft tumours over expressing EGFR (Goldstein J et al. J Immunol 1997; 158:872-9).

Phase I clinical trials in patients with advanced EGFR-expressing solid tumors have demonstrated that Cetuximab is well tolerated (Robert F et al. J Clin Oncol 2001; 19:3234-43*;* Baselga J et al. J Clin Oncol 2000; 18:904-14; Shin DM et al. Clin Cancer Res 2001; 7:1204-13). The most clinically relevant adverse events attributable to Cetuximab were allergic reactions and skin toxicity (Shin DM et al. Clin Cancer Res 2001; 7:1204-13). Cetuximab has recently approved by US Food and Drug Administration (FDA), either monotherapy or in combination with irinotecan, for the treatment of advanced colon rectal cancer patients with detectable EGFR expression *(ImClone Systems, Erbitux (Cetuximab). US Prescribing IFNormation. ImClone System, 2004).* In addition, extensive phase II and III clinical testing of Cetuximab has continued in pancreatic carcinoma patients (Xiong HQ et al. J Clin Oncol 2004; 22:2610-6*)*, non-small cell lung cancer (NSCLC) patients (Lynch TJ et al. Proc Am Soc Clin Oncol 2004*;* Rosell R et al. Proc. Am. Soc Clin. Oncol. 2004*)* and squamous cell carcinoma of the head and the neck (SCCHN) patients (Bonner JA et al. Proc. Am. Soc. Clin. Oncol. 2004).

Also, the humanized antibody h-R3/TheraCIM (Center of Molecular Immunology) is been evaluated. This Mab has a similar capacity to original murine antibody to inhibit EGFR/EGF binding (Mateo C et al. Immunotechnology 1997; 3:71-81*).* The h-R3 capacity to inhibit the proliferation of A431 cell line in monolayer was similar to Cetuximab. In pre-clinical studies using h-R3, have obtained complete regressions of human tumor xenografts overexpressing EGFR (Viloria-Petit A et al. Cancer Res 2001; 61:5090-101). h-R3 was registered in Cuba by Center for Drug Quality Control (CECMED) for the treatment of advanced head and neck cancer patients (Crombet T et al. J Clin Oncol 2004; 22:1646-54). Also, clinical trials testing of h-R3 has continued in other localizations such as: brain, breast, prostate and lung (*Crombet T, personal communication*).

Other anti-EGFR Mabs that have a similar mechanism of action are currently under clinical investigation. ABX-EGF is fully human IgG2 anti-EGFR Mab that inhibits ligand-dependent receptor activation and inhibits the growth of human tumor xenografts (Yang X et al. Cancer Res 1999; 59:1236-43). Recently, positive results of III phase clinical testing of ABX-EGF in colon rectal cancer patients have been reported *(*Tyagi P. Clin Colorectal Cancer 2005; 5:21-3). Moreover, II phase clinical trials with this Mab in renal cancer and NSCLC patients are ongoing (Tiseo M et al. Curr Med Chem Anticancer Agents 2004; 4:139-48). EMD 72000 (humanized anti-EGFR Mab) is been evaluated in pancreatic cancer patients (Graeven U et al. Br J Cancer 2006; 94:1293-9). However, the metastatic cancer patient treated with the anti-EGFR Mabs have not reached significant survival benefits. For example, the irinotecan-refractory colon rectal cancer patients treated with Cetuximab had illness stabilization but they did not reach an increase of survival *(*Cunningham D et al. N. Engl. J. Med. 2004; 351: 337-345). These results lead to the search of therapeutic combinations that allow increasing anti-EGFR Mab efficacy.

### Cytotoxic T lymphocytes induction by Mab-based passive therapy

Cross-priming was first described by Michael Bevan more than 25 years ago (Bevan MJ. J Exp Med 1976; 143: 1283-88). This phenomenon is based in the ability of the antigen-presenting cells (APC) to prime cytotoxic T lymphocytes (CTL) responses against minor histocompatibility antigens captured from foreign donor cells. A number of factors have been identified to promote and improve the delivery of antigen to the MHC class-I presentation pathway of dendritic cells (DC). Among those are heat-shock proteins (Suto R, Srivastava PK. Science 1995; 269: 1585-88*),* exosomes (Wolfers J et al. Nature Medicine 2001: 7: 297-303), and immune complexes (Regnault A et al. J Exp Med 25 1999; 189: 371-80). Dying cells, apoptotic or necrotic, are an especially attractive source of antigen for cross-presentation. The immunological consequences of the ingestion of apoptotic/necrotic cellular material by DC are controversial *(*Russo V et al. PNAS 2000; 97: 2185-90*;* Yrild BU et al. J Exp Med 2000; 191: 613-21*).* In general, necrotic cell material is considered to be immunogenic, while apoptosis is thought to be immunologically innocuous or even tolerizing. However, in certain model systems apoptotic cell death has been shown to be an attractive immunogenic antigenic source for the cross-priming of CTL because it release "danger signals" for APC maturation (Lake RA, Robinson BWS. Nature Reviews 2005; 5: 397-405).

C2B8 (Rituximab) is a chimeric mouse-human MAb against CD20 *(*Relf Meet et al. Blood 1994; 83: 435-45). This agent is used in the treatment of non-Hodgkin's lymphomas of the B-cell type where it promotes a rapid and efficient depletion of normal and neoplastic B cells with a response rate of about 50% and progression-free intervals of the disease up to 12 months (Maloney DG. Curr Opin Haematol 1998; 5:237-43*;* Coiffier B et al. Blood 1998; 92:1927-32*;* Hainsworth JD et al. Blood 2000; 95:3052-56*).* Several studies have indicated that maximal clinical and molecular responses to Rituximab therapy may take several months, suggesting that short-term cytolytic mechanisms such as apoptosis, complement-dependent cytotoxicity (CDC), and ADCC are not the only ones involved. Rituximab promoted lysis of lymphoma cells through any of these latter mechanisms may promote uptake and cross-presentation of lymphoma cell-derived peptides by DC, inducing their maturation and allowing the generation of specific CTL (Selenko N et al. J. Clin. Oncol 2002; 3:124-130). The "vaccine effect" induced by Rituximab has not been strictly studied. Randomized clinical trials are needed to confirm the clinical impact of this approach.

### Alpha/ beta type I Interferons as anti-tumor therapy

Alpha/beta type I IFNs (IFNs-α/β) are biological agents used for the anti-cancer therapy, specifically in melanoma and renal carcinoma patients *(*Agarwala SS, Kirkwood JM. Semin. Surg. Oncol. 1998; 14: 302-310*;* Vlock DR et al. J. Immunother. Emphasis Tumor Immunol. 1996; 19:433-442*;* Kirkwood JM et al. Semen. Oncol. 1997; 24: 16-23*;* Kirkwood JM et al. J Clin. Oncol 1996, 14: 7-17*).* IFN-α was the first cytokine produced by recombinant DNA technology, it has demonstrated to regulate the proliferation and tumor differentiation (Hertzog et al. J Biol Chem 1994; 269:14088-93). Also, it has been reported its effect in apoptosis induction *(*Clemens MJ. J Interferon Cytokine Res 2003; 23:277-92*)* and angiogenesis inhibition *(*Sidky YA et al. Cancer Res 1987; 47:5155- 61*).* In addition to INFs-α/β effect on tumor cells, INFs-α/β display a several effects on host immune cells, which can play an important role in the anti-tumor immune response (Belardelli F. APMIS. 1995; 103:161-179). However, the data from IFN-α clinical effectiveness in solid tumors are inconsistent. In fact, only patients with specific tumors are benefited, while others are partial or totally resistant to this therapy.

Studies about the INF-α role in the regulation of EGFR expression on tumor cells have been published (Budillon A et al. Cancer Res 1991; 51: 1294-9*;* Caraglia M et al. Int J Cancer 1995; 4: 309-16*;* Heise H et al. Anti Cancer Drugs 1995, 6; 686-92*;* Scambia G et al. Int J Cancer 1994; 58: 769-73*;* Yang JL et al. Gut 2004; 53: 123-129*;* Qu XJ et al. J Urology 2004; 172: 733-738*).* Based in the reports that demonstrates the INF-α capacity to increase the expression and EGFR activity in some tumors, different authors have studied the combination of EGFR tyrosine kinase inhibitors (EGFR TKls) and INF-α obtaining an anti-tumor advantage for the combined therapy (Yang JL et al. Oncology 2005; 69: 224-238*;* Brúcese Fet al. Clin Cancer Res 2006; 12: 617-625*;* Yang JL et al. Cancer Letters 2005; 225: 61-74). Nevertheless, these results can not be generalized because the IFN-α effect on EGFR expression in tumor cells is very variable *(*Scambia G et al. Int JCancer 1994*;* Yang JL et al. Gut 2004; 53: 123-129*;* Qu XJ et al. J Urology 2004; 172: 733-738*).* This phenomenon could limit the advantage of the INF-α treatment for a patient niche. On the other hand, IFN-α can increase the mayor histocompatibility complex class I (MHC I) molecules in normal tissues *(*Cho HJet al. J Immunology 2002; 168: 4907-13*;* Lang KS et al. Nature Medicine 2005; 11: 138-44). In this invention, it is shown that the INF-α application to tumor cells can increase MHC I expression, even if the tumors decrease MHC I molecules as escape mechanisms to immunologic effectors. Consequently, the IFN-α/anti-EGFR Mab combination could be more advantageous than IFN-α/EGFR TKI combination due to the anti-EGFR Mabs could induce CTL response and this effect do not described to EGFR TKls.

The present invention is based on two biological events neither described nor suggested by the previous art. Firstly, the anti-EGFR Mab-based therapy is CD8⁺ T cells dependent. Specifically, the anti-metastatic effect of anti-EGFR Mabs is CD8⁺ T cells dependent. Secondly, type I IFN treatment of tumor cells increases MHC I expression. The combined application of these facts allows outstandingly increasing the anti-cancer therapeutic effect of the anti-EGFR Mabs and the type I IFNs.

### Detailed disclosure of the invention.

The present invention relates to a therapeutic composition comprising humanized anti-EGFR Mabh-R₃ and type I IFNs (one or several), a humanized for use in the treatment of cancer, wherey the antibody and IFNs are simultaneously or sequentially administered. Moreover, the therapeutic composition of the present invention comprises type I IFNs, and more particularly the composition comprises IFN-α, and more specifically the recombinant human IFN-α.

In another embodiment, the present invention relates to a pharmaceutic kit composed by a container with the anti-EGFR Mab as defined above, one or several containers with one or several IFNs and a label or other instructions to dosage and use.

Due to ethical reasons it is impossible the experimentation in human being so the present invention further relates to an experimental model to demonstrate "in vivo" the technical solution disclosed by the present invention. This experimental model comprises a murine antibody against the murine EGF receptor as well as the biological effect of this antibody on the growth of the tumor cell lines.

### Evaluation of the anti-metastatic effect of anti-EGFR Mab treatment

Balb/c or C57BL/6 mice, aged 8-12 weeks, are used as experimental model for the evaluation of the anti-metastatic effect of anti-EGFR Mab treatment.

Mice are treated with a Mab specific for the extracellular domain of murine EGFR or a control Mab (antibody with the same isotype of anti-EGFR Mab, which be irrelevant to each tumor) using dose between 1 and 25 mg/kg. The antibodies are inoculated by intravenous or intraperitoneal injection. The administration protocol can be conducted by different ways:
- To begin the day before tumor challenge and to continue days 1, 2, 3 after the tumor challenge. After the 6^{th} day, the treatment is reinitiated with three doses per week until the end of assay.
- To begin the 2^{nd} day after tumor challenge and an additional dose the 3^{rd} day. After the 6^{th} day the administration is reinitiated with three doses per week until the end of assay.
- To begin 6th day after tumor challenge with three doses per week until the end of assay.

The murine tumor cells that express EGFR (from lung, breast, colon, prostate, brain, bladder and head & neck tumors) are inoculated in mice at day zero. The amounts of tumor cells inoculated are between 1 x 10³ and 1 x 10⁶ per mouse. The tumor cells can be administered by intravenous, subcutaneous or intramuscular injection to obtain lung or liver metastasis. Mice are sacrificed by cervical dislocation (20 to 45 days after tumor challenge). The metastases for each organ are counted using a stereoscopic microscope.

### Measuring of CD8⁺ T cells role in the anti-metastatic effect of anti-EGFR Mab

Mice are inoculated with the tumor cells as described previously (day 0).They receive intravenous or intraperitoneal injections of a Mab specific for CD8 molecule, which is able to eliminate CD8 positive cells (5-50 mg/Kg). The anti-CD8 Mab administration begins the day -1 to 6 and continues every four days until the end of the assay. Also, mice are treated with an anti-EGFR Mab as describe previously. Mice are sacrificed by cervical dislocation (20 to 45 days after tumor challenge). The metastases for each organ are counted using a stereoscopic microscope.

### Evaluation of the anti-metastatic effect of IFN-α/anti-EGFR Mab combination

Mice are inoculated with the tumor cells and anti-EGFR Mab as described previously. Moreover, these mice are treated with murine IFN-α (5 x 10⁵- 5 x 10⁶ U/Kg) by intravenous, intraperitoneal or subcutaneous injection. The administration protocol can be conducted by different ways: (a) anti-EGFR Mab and IFN-α simultaneously, (b) pre-treatment (IFN-α) and treatment (anti-EGFR Mab) or (c) pre-treatment (IFN-α) and treatment (anti-EGFR Mab + IFN-α). Mice are sacrificed by cervical dislocation (20 to 45 days after tumor challenge). The metastases for each organ are counted using a stereoscopic microscope.

### Immunotherapeutic composition comprising anti human EGF-R antibodies and α-INF.

The composition of the present invention comprises the passive immunotherapy with specific MAbs against the extracellular domain of the human EGF-R together with α-INF will be administered to patients immediately after diagnosis and/or surgical treatment. The composition of the present invention should induce CD8+ T cells-based immune response in those individuals under treatment.

The therapeutically composition comprising the anti EGF-R antibody and the α-INF has a synergistic effect on the lung metastasis development.

The procedure consists of administering to patients bearing advanced cancer of epithelial origin a dose between 100 to 400 mg of an anti-EGFR MAb and the human recombinant α-INF in a dose between 10 - 30 x 10⁶ IU/ m²/day. The injections could follow several schedules. Preferably, the therapeutic composition of the present invention follows any of the following schedules: (a) a monthly injection during one week or (b) four consecutives weeks every three months. The treatment will continue until partial or complete tumor regression, or until any adverse reaction occurs that requires treatment cessation.

### Examples:

### Example 1: Obtaining an anti-murine EGFR Mab.

Balb/c mice were immunized with a recombinant protein of the extracellular domain of murine EGFR (Sánchez B et al. Int J Cancer 2006; 119:2190-2199) emulsified in Freund's adjuvant. Sera were processed at day 0 and 60. The specific antibodies against the protein recombinant were measured by ELISA. Inoculated mice development high serum IgG levels (1:80 000-1:100 000) against the recombinant protein. A mouse showing the highest antibody titer against the recombinant protein was selected for the fusion experiment. A Mab specific for the extracellular domain of murine EGFR, 7A7 (IgG1), was obtained *(*Garrido G et al. Hybridoma and Hybridomics 2004; 23 (3): 168-175*)*. This Mab specifically recognize the murine EGFR present in tumor cells by different techniques, such as: Western Blot, FACS and immunohistochemistry.

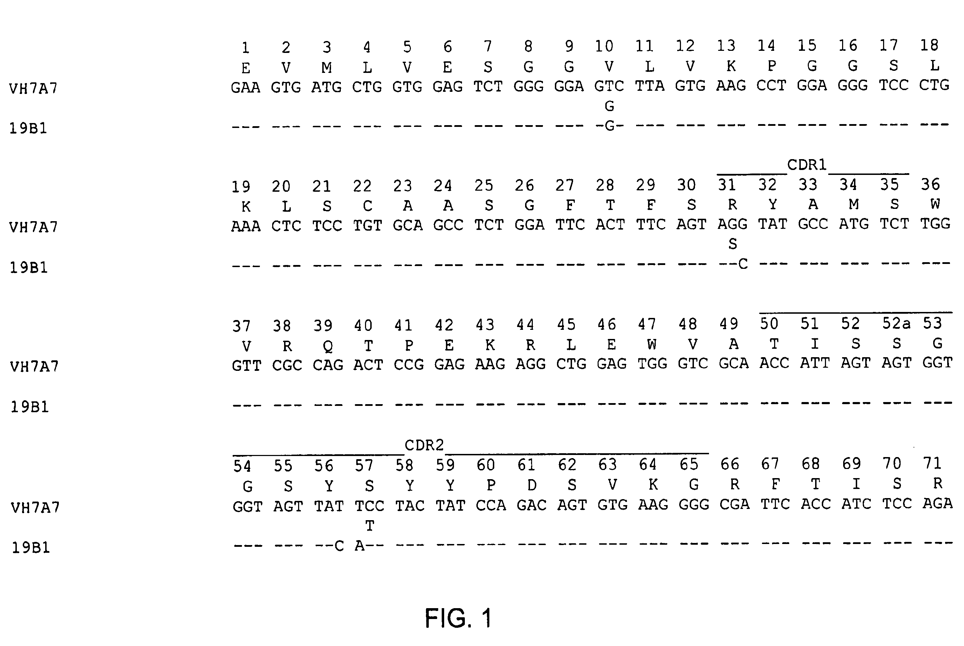

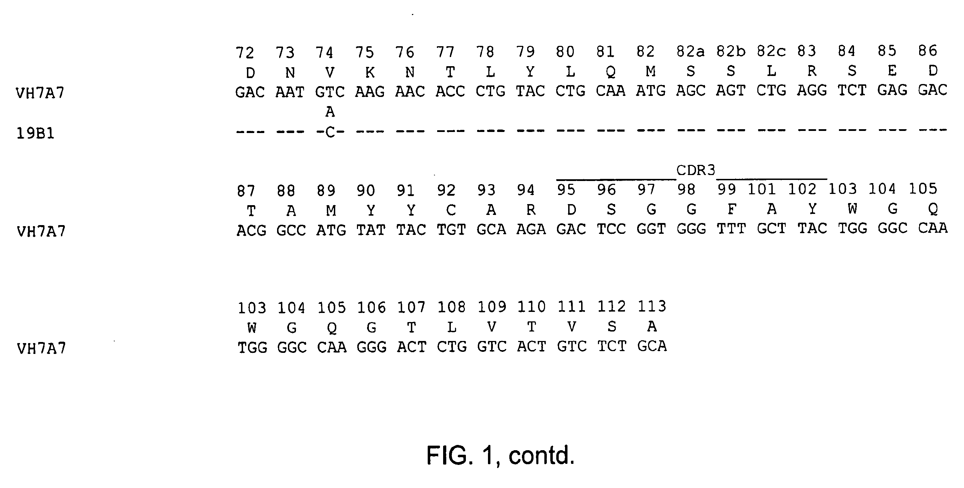

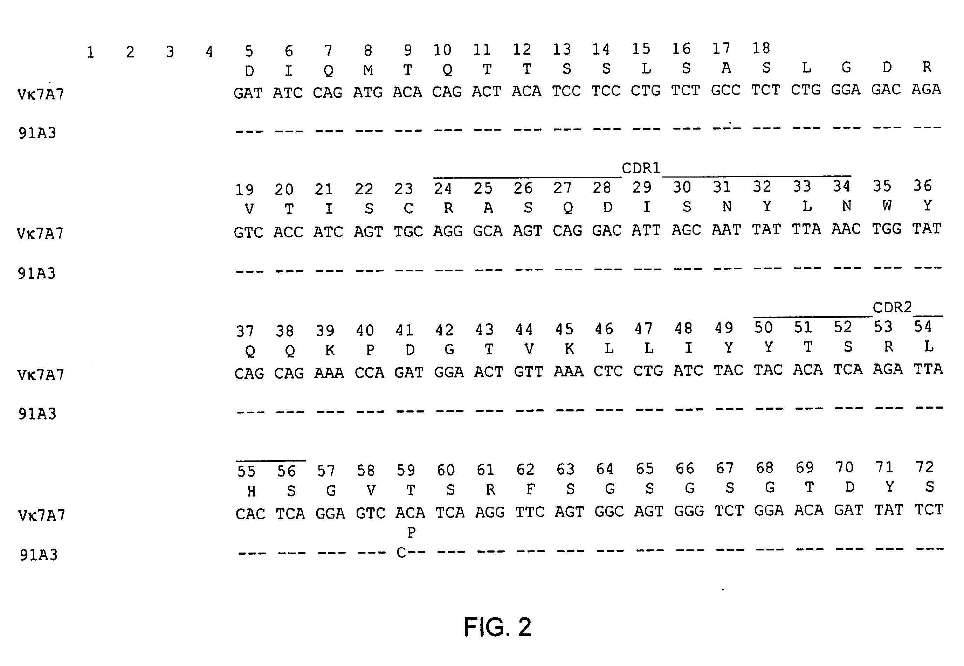

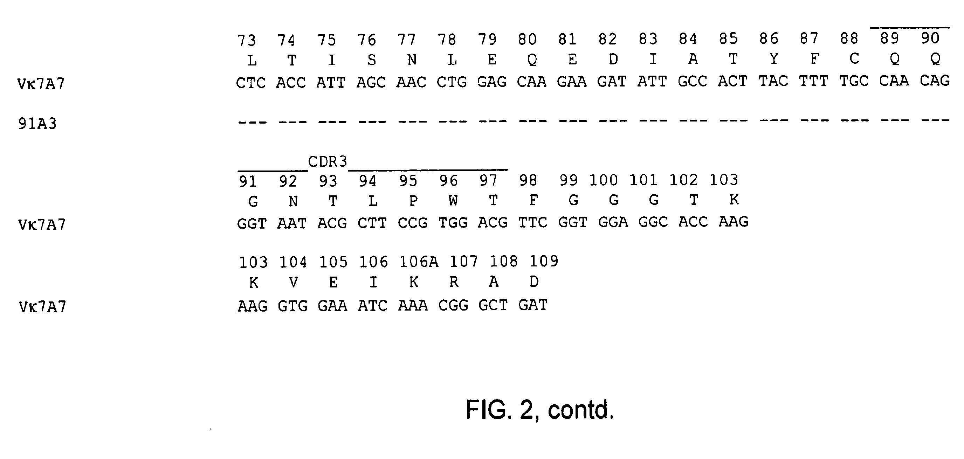

The nucleotide sequence and the deduced amino acid sequence of the heavy chain variable region of 7A7 Mab (GenBank access number: DQ437656) are shown in Figure 1. The nucleotide and deduced amino acid sequences of light chain variable region (Vκ) of 7A7 Mab (GenBank access number: DQ437657) are shown in Figure 2.

### Example 2: 7A7 Mab anti-metastatic effect on D122 tumor.

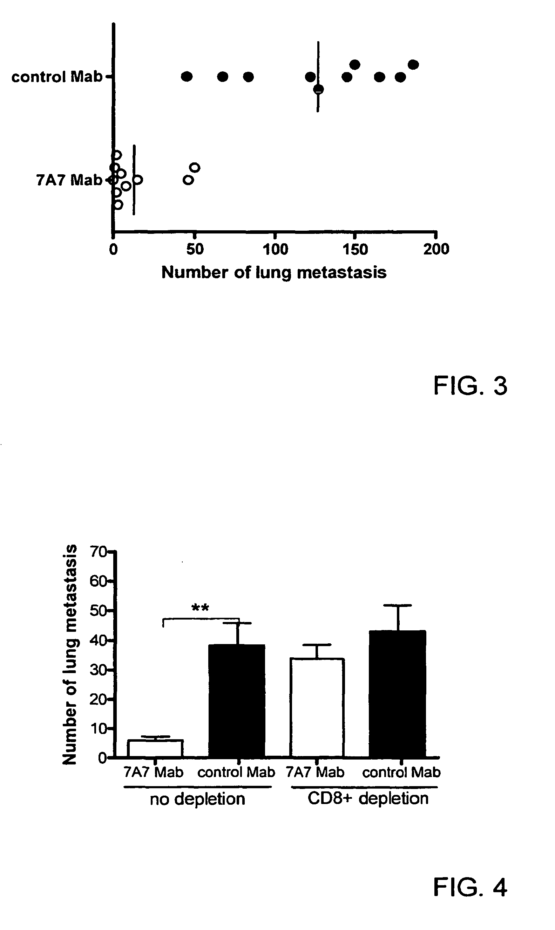

D122 cells (2.5 x 10⁵) [D122 tumor is metastatic clone of the Lewis lung carcinoma] were injected into lateral tail veins of C57BL/6 mice. 7A7 and control Mab (28 mg/kg in 100 µl PBS) were administered the day six after tumor challenge and continued three doses per week. Three weeks after tumor injection, the mice were sacrificed, and the lungs were removed. The number of D122 lung metastasis was counted. Administration of 7A7 Mab significantly reduced the number of D122 lung metastasis compared with a control Mab (Figure 3), this difference was significant statistically (Mann-Whitney test, p < 0.0001).

### Example 3: 7A7 Mab anti-metastatic effect on D122 tumor is dependent of CD8⁺ T cells.

D122 cells (2.5 x 10⁵) were injected into lateral tail veins of C57BL/6 mice. 7A7 and control Mab (28 mg/kg in 100 µl PBS) were administered the day six after tumor challenge and continued three doses per week. Depletion of CD8⁺ cells by a specific antibody (intraperitoneal injection) began the day six after tumor challenge and continued until the end of assay. The effectiveness of depletions was assessed in the spleen and the lung of mice. Three weeks after tumor injection, the mice were sacrificed, and the lungs were removed. The number of D122 lung metastasis was counted.

In this experiment, 7A7 Mab anti-metastatic effect on D122 tumor was verified, being observed a significant reduction in the number of D122 lung metastasis in the 7A7-treated mice compared with the control mice (Dunn test, p < 0.01) (Figure 4/Table 1). CD8⁺ cell depletion abrogated of 7A7 Mab anti-metastatic effect, obtaining a median of lung metastasis number for the 7A7 group greater than the median of lung metastasis number for the control group (Dunn test, p < 0.05) (Figure 4/Table 1).

**Table 1. Metastasis number median of the experimental groups.**

| **Median** | | | |
|---|---|---|---|
| **7A7 Mab** | **Control Mab** | **747 Mab+ depletion** | **control Mab + depletion** |
| 7 | 44 | 49 | 44 |

### Example 4: MHC I levels increased in D122 and MB16F10 cells by IFN-α treatment.

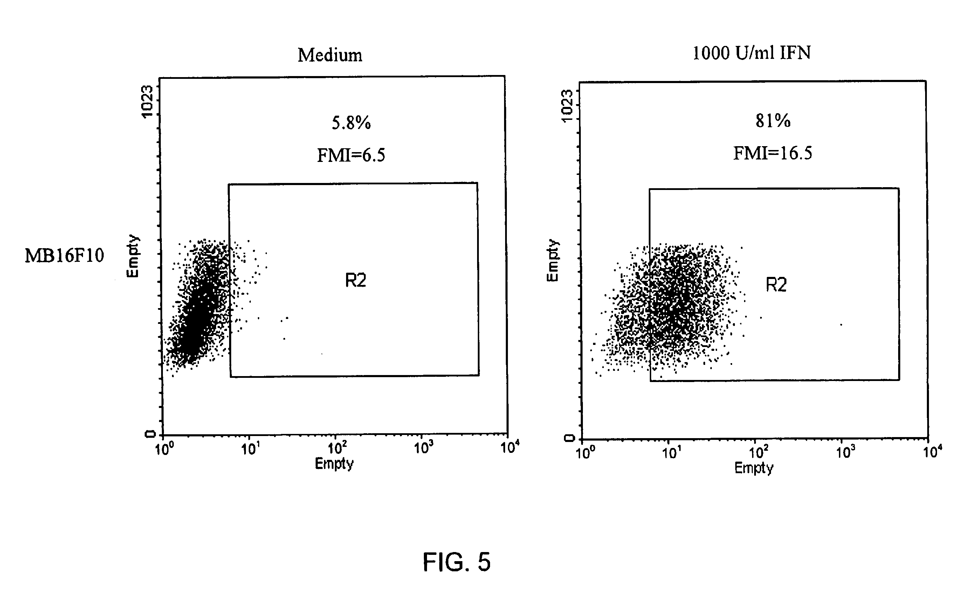

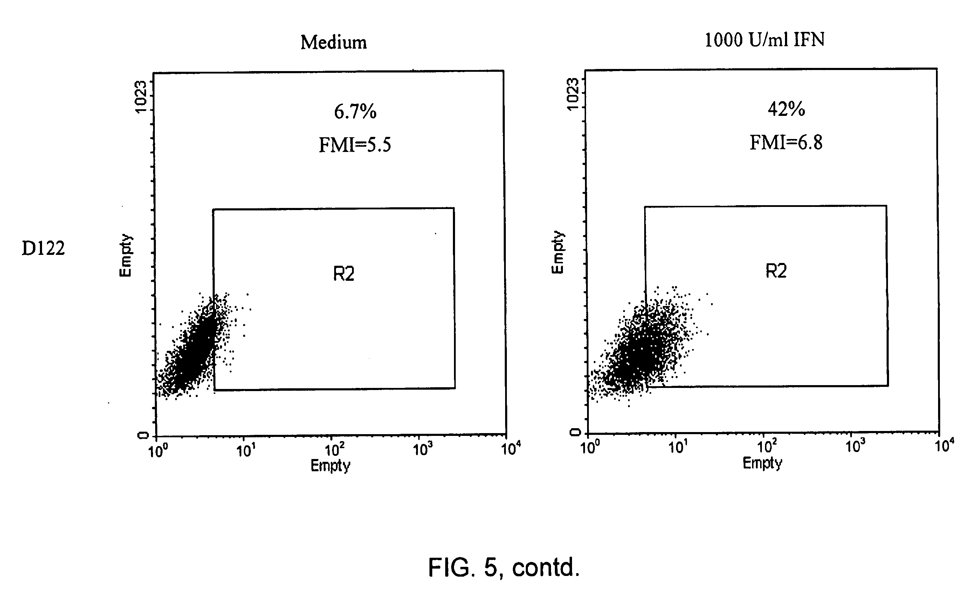

D122 and MB16F10 cells (0.25 x 10⁶/ 6-well plate) were treated with IFN-a (1000 U/ml) for 12 hours. Next, MHC I expression level on cell membrane was determined in treated and non-treated cells by FACS. Cells (2 x 10⁵) were incubated in PBS containing 0.1% NaN₃ and 1% BSA (B solution) for 15 min at 4°C. Subsequently, the cells were stained with a Mab specific for the H-2kb molecule diluted in B solution (1:200, Pharmingen, EEUU). After washing, 10⁴ cells were acquired using a FACScan flow cytometer (Becton Dickison). The data obtained were analyzed using WinMDI software (version 2.8). The IFN-α treatment provoked an increase of MHC I expression in the membrane of D122 and MB16F10 cells, this treatment also increased the percentage of IFN-α positive cells (Figure 5).

### Example 5: The effect of the α-IFN treatment on the EGFR expression in D122 cells.

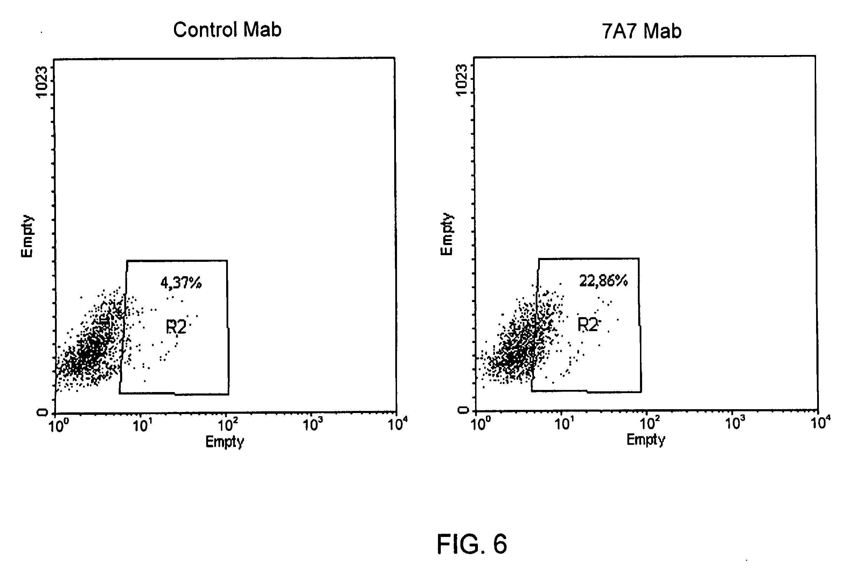

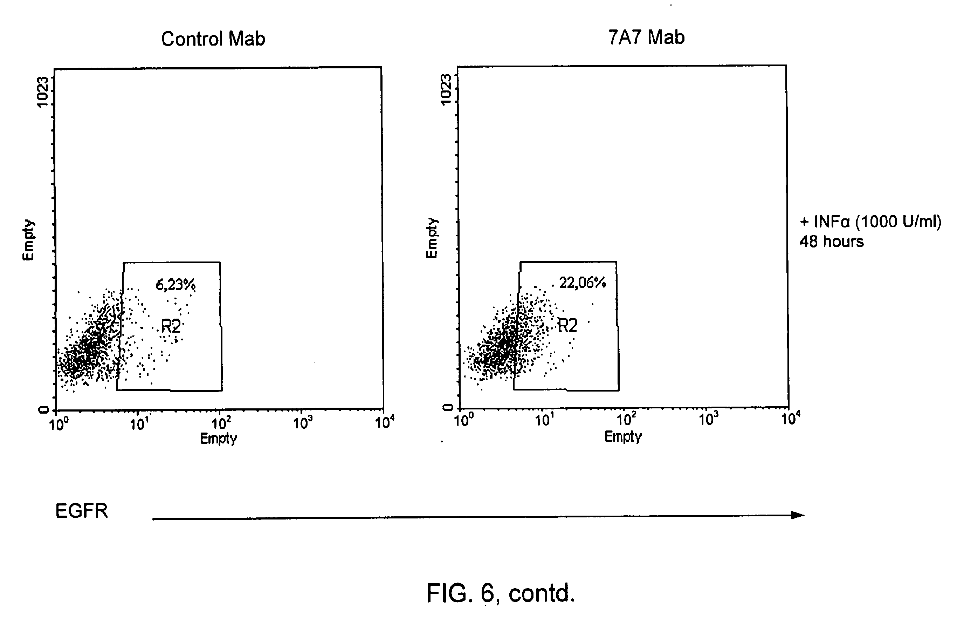

D122 cells (0.25 x 10⁶/ 6-well plate) were treated with IFN-α (1000 U/ml) for 48 hours. Next, EGFR expression level on cell membrane was determined in treated and non-treated cells by FACS. Cells (2 x 10⁵) were incubated in PBS containing 0.1% NaN₃ and 1% BSA (B solution) for 15 min at 4°C. Subsequently, the cells were stained with 7A7 Mab (1 µg/ml) diluted in B solution for 15 min at 4°C. After washing, a goat antimouse total lgs FITC conjugated was added (1:200; Pharmingen, EEUU). After washing, 10⁴ cells were acquired using a FACScan flow cytometer (Becton Dickison). The data obtained were analyzed using WinMDI software (version 2.8). IFN-α treatment of D122 cells did not change the EGFR expression (Figure 6).

### Example 6: Anti-metastatic effect of the 7A7 Mab/ α-IFN combination on D122 tumor.

D122 cells (2.5 x 10⁵) were injected into lateral tail veins of C57BL/6 mice (10 mice per group). The co-administration of IFN-α (5 X 10⁵ U/Kg, intraperitoneal injection) and 7A7 Mab (1 mg/kg, intravenous injection) began the day six after tumor challenge and continued three times per week until the end of assay. Three weeks after tumor injection, the mice were sacrificed, and the lungs were removed. The number of D122 lung metastasis was counted. Mice treated with PBS or 7A7 Mab or α-IFN- were used as control.

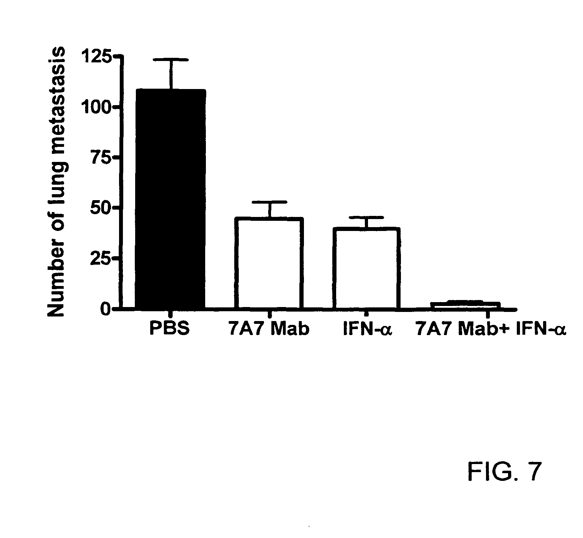

In this experiment, 7A7 Mab and α-IFN-anti-metastatic effects on D122 tumor were verified (as monotherapy), being observed a reduction in the number of D122 lung metastasis in the 7A7-treated mice and α-IFN-treated mice compared with the PBS-treated mice (Figure 4/Table 1). However, this anti-metastatic effect was significantly increased when mice received the combined therapy (Figure 7[Table 2) (PBS vs. AcM 7A7+α-IFN: p< 0,001; AcM 7A7 vs AcM 7A7+α-IFN: p< 0,05; α-IFN vs AcM 7A7+α-IFN: p< 0,05, Dunn test).

**Table 2. Metastasis number median of the experimental groups.**

| **Median** | | | |
|---|---|---|---|
| **PBS** | **7A7 Mab** | **α-IFN** | **7A7 Mab+α-IFN** |
| **103** | **42** | **36** | **2** |

### Brief description of the drawings:

**Figure1****.** Nucleotide and deduced amino acid sequences of the cDNA encoding the heavy variable region of 7A7 Mab. The amino acids are enumerated according to Kabat. Spaces have been introduced to maximize alignment. The amino acids residue encoded by each codon is given above the nucleotide sequence.
**Figure 2****.** Nucleotide and deduced amino acid sequences of the cDNA encoding the light variable region of 7A7 Mab. The amino acids are enumerated according to Kabat. Spaces have been introduced to maximize alignment. The amino acids residue encoded by each codon is given above the nucleotide sequence.
**Figure 3****.** 7A7 Mab anti-metastasic effect on D122 tumor. C57BL/6 mice were inoculated with D122 cell (experimental metastasis model) and treated with 7A7 or control Mab. Three weeks after tumor injection, the mice were sacrificed, and the lungs were removed. The number of D122 lung metastasis was counted.
**Figure 4****.** 7A7 Mab anti-metastatic effect on D122 tumor is dependent of CD8⁺ T cells. C57BL/6 mice were inoculated with D122 cell (experimental metastasis model) and treated with 7A7 or control Mab. Mice were depleted of CD8 positive cell populations using an anti-CD8 Mab. Three weeks after tumor injection, the mice were sacrificed, and the lungs were removed. The number of D122 lung metastasis was counted.
**Figure 5****.** MHC I levels increased in D122 and MB16F10 cells by IFN-α treatment. D122 and MB16F10 cells were treated with IFN-α for 12 hours. Finally, the cells were incubated with a Mab specific for the H-2kb molecule FITC conjugated. The percentage of H-2kb positive cells was measured by FACS.
**Figure 6****.** The IFN-α treatment does not change EGFR expression on D122 cells. D122 cells were treated with α-IFN for 48 hours. Finally, the cells were incubated with 7A7 Mab. The percentage of EGFR positive cells was measured by FACS.
**Figure 7****.** The anti-metastatic effect of the combined treatment 7A7 Mab/α-IFN was superior to the independent treatments. C57BL/6 mice were inoculated with D122 cell (experimental metastasis model) and treated with 7A7 and α-IFN-. Three weeks after tumor injection, the mice were sacrificed, and the lungs were removed. The number of D122 lung metastasis was counted.

## Claims

1. A therapeutic composition comprising humanized anti-EGFR Mab h-R3 and one or several type I interferons for use in a method for the treatment of cancer, whereby the humanized anti-EGFR Mab h-R3 and the one or several type I interferons are simultaneously or sequentially administered.

2. The therapeutic composition according to claim 1, further comprising an appropriate exicipient for the intravenous injection.

3. The therapeutic composition according to claim 1 or 2, wherein the interferon is human α-interferon.

4. The therapeutic composition according to claim 3 wherein the interferon is human recombinant α-interferon.

5. The therapeutic compositions according to claims 1 to 4, comprising the sequential administration of the antibody and the interferon.

6. The therapeutic composition according to claims 1 to 5, wherein the cancer is an advanced cancer of epithelial origin, and the humanized anti-EGFR Mab h-R3 is provided in a dose of between 100 to 400 mg, and the human recombinant α-INF in a dose of between 10 - 30 x 106 IU/ m2/day.

7. A pharmaceutical kit composed by a container with humanized anti-EGFR Mab h-R3, one or several containers with one or several IFNs and a label or additional instructions to dosage and use.

## Patentansprüche

1. Therapeutische Zusammensetzung, umfassend humanisierten Anti-EGFR Mab h-R3 und ein oder mehrere Typ-I-Interferone zur Verwendung in einem Verfahren zur Behandlung von Krebs, wobei der humanisierte Anti-EGFR Mab h-R3 und das eine oder die mehreren Typ-I-Interferone gleichzeitig oder sequenziell verabreicht werden.

2. Therapeutische Zusammensetzung nach Anspruch 1, ferner umfassend einen geeigneten Hilfsstoff zur intravenösen Injektion.

3. Therapeutische Zusammensetzung nach Anspruch 1 oder 2, wobei das Interferon humanes α-Interferon ist.

4. Therapeutische Zusammensetzung nach Anspruch 3, wobei das Interferon humanes rekombinantes α-Interferon ist.

5. Therapeutische Zusammensetzung nach den Ansprüchen 1 bis 4, umfassend die sequenzielle Verabreichung des Antikörpers und des Interferons.

6. Therapeutische Zusammensetzung nach den Ansprüchen 1 bis 5, wobei der Krebs ein fortgeschrittener Krebs von epithelialem Ursprung ist und der humanisierte Anti -EGFR Mab h-R3 in einer Dosis zwischen 100 bis 400 mg und das humane rekombinante α-INF in einer Dosis zwischen 10-30 x 106 IU/m2/Tag bereitgestellt werden.

7. Pharmazeutisches Kit, zusammengesetzt aus einem Behälter mit humanisiertem Anti-EGFR Mab h-R3, einem oder mehreren Behältern mit einem oder mehreren IFNs und einem Aufkleber oder zusätzlichen Anweisungen für Dosierung und Verwendung.

## Revendications

1. Composition thérapeutique comprenant l'anticorps monoclonal anti-EGFR humanisé h-R3 et un ou plusieurs interférons de type I destinée à être utilisée dans un procédé de traitement du cancer, l'anticorps monoclonal anti-EGFR humanisé h-R3 et un ou plusieurs interférons de type I étant administrés simultanément ou successivement.

2. Composition thérapeutique selon la revendication 1, comprenant en outre un excipient approprié pour une injection intraveineuse.

3. Composition thérapeutique selon la revendication 1 ou la revendication 2, dans laquelle l'interféron est un interféron α humain.

4. Composition thérapeutique selon la revendication 3, dans laquelle l'interféron est un interféron α humain recombinant.

5. Composition thérapeutique selon les revendications 1 à 4, comprenant l'administration successive de l'anticorps et de l'interféron.

6. Composition thérapeutique selon les revendications 1 à 5, dans laquelle le cancer est un cancer d'origine épithéliale avancé, et l'anticorps monoclonal anti-EGFR humanisé h-R3 est administré à une dose comprise entre 100 et 400 mg, et l'INF-α humain recombinant à une dose de 10 à 30 x 10⁶ UI/m²/jour.

7. Kit pharmaceutique composé d'un récipient contenant l'anticorps monoclonal anti-EGFR humanisé h-R3, d'un ou plusieurs récipients contenant un ou plusieurs interférons et d'une étiquette ou d'une notice supplémentaire relative à la posologie et à l'utilisation.
